# EUROPÄISCHE PATENTANMELDUNG

(11) **EP 0 972 497 A2**
(43) Veröffentlichungstag der Anmeldung: **19.01.2000**
(21) Anmeldenummer: 99112690.5
(22) Anmeldetag: 02.07.1999
(51) Int. Cl.: A61F 2/04, F16K 15/14

(54) **Rückschlagventil, insbesondere für eine implantierbare künstliche Harnblase**

(30) Priorität: 15.07.1998 DE 19831698
(71) Anmelder: CareMed Medical Produkte Aktiengesellschaft, 01109 Dresden (DE)
(72) Erfinder: Gerlach, Roland, Dr.-Ing., 34302 Guxhagen (DE); Hannappel, Josef, Prof. Dr. med., 50259 Pulheim (DE); Reuter, Jürgen, Dipl. Ing., 36211 Alheim (DE); Rohrmann, Dorothea, Dr. med., 52379 Langerwehe (DE)
(74) Vertreter: Dallmeyer, Georg, Dipl.-Ing.

(57) **Zusammenfassung**

Das Rückschlagventil weist einen Einlaßkanal (31), einen Auslaßkanal (32) und eine dazwischen angeordnete Ventilkammer (34) auf. Die Ventilkammer (34) steht über Öffnungen (35) ständig mit dem Einlaßkanal (31) in Verbindung. Auf einem Blindstutzen (33) ist ein Ventilorgan (35) befestigt, das aus einem elastischen formstabilen Schlauch besteht. Das Ventilorgan (36) erstreckt sich bis in den Auslaßkanal (32) hinein und liegt an der Wand des Auslaßkanals an. Bei einem Druck, der den Öffnungsdruck des Ventils übersteigt, hebt das Ventilorgan (36) von der Wand des Auslaßkanals (32) ab.

## Beschreibung

Die Erfindung betrifft ein Rückschlagventil, insbesondere für eine implantierbare künstliche Harnblase, das einerseits einen Rückfluß verhindert, andererseits aber auch einen definierten Öffnungsdruck hat.

Es gibt Fälle, in denen Rückschlagventile als Implantate in den menschlichen Körper eingepflanzt werden. Aus der Zeitschrift "The Journal of Urology", Vol. 151, 1996, 2094-2097, ist eine künstliche Harnblase bekannt, die zwei selbstrückstellende Implantatbehälter aufweist, welche einerseits über Katheter mit den Nieren und andererseits über ein Y-Stück mit der Harnröhre verbunden sind. Die Behälter, die bestrebt sind, sich aufzuweiten, nehmen aus den Nieren Flüssigkeit auf. Zum Ablassen der Flüssigkeit drückt der Patient auf die Behälter, so daß diese sich in die Harnröhre entleeren. Jeder der Behälter ist an seinem Einlaß und an seinem Auslaß mit einem Rückschlagventil versehen, um einerseits einen Reflux in die Niere und andererseits ein Ansaugen von Luft während der Füllungsphase zu vermeiden. Bei diesen Rückschlagventilen handelt es sich um Folienventile, die auch als "Entenschnabelventile" bezeichnet werden. Solche Folienventile haben einen sehr niedrigen Öffnungsdruck, so daß die Gefahr des unbeabsichtigten Entleerens der Behälter, und somit die Gefahr der Inkontinenz, besteht. Außerdem neigen implantierte Folienventile zur Ansammlung von Inkrustationen und somit zu Undichtigkeiten.

Künstliche Harnblasen mit entsprechenden Ventilen sind ferner beschrieben in EP 0 282 157 B1 und EP 0 393 714 A2. Hierbei sind relativ komplexe Rückschlagventile vorgesehen, bei denen die Gefahr des Versagens nach langer Implantationszeit besteht. DE 27 60 437 beschreibt eine prothetische Blase. Die in die Blase hineinführenden Harnleiterkatheter sind mit Folienventilen versehen.

Schließlich ist aus US 4 722 731 ein Ventil bekannt, das ein Ventilorgan in Form eines flexiblen Schlauchs aufweist, in den von einem Ende her ein Einlaßkanal und vom anderen Ende her ein Auslaßkanal hineinragt. Der flexible Schlauch ist so vorgespannt, daß er die Verbindung zwischen dem Einlaßkanal und dem Auslaßkanal versperrt. Wenn der Öffnungsdruck ein vorgegebenes Maß übersteigt, öffnet das Ventil. Auch ein derartiges Überdruckventil hat einen komplexen Aufbau und enge Kanäle, die von der Flüssigkeit durchströmt werden müssen.

Der Erfindung liegt die Aufgabe zugrunde, ein Rückschlagventil zu schaffen, das sehr einfach aufgebaut ist und ein definiertes Öffnungsverhalten aufweist.

Die Lösung dieser Aufgabe erfolgt erfindungsgemäß mit den im Patentanspruch 1 angegebenen Merkmalen.

Das erfindungsgemäße Rückschlagventil zeichnet sich dadurch aus, daß das Ventilorgan ein an einem Ende offener, elastischer formstabiler Schlauch ist, an dem der Fließweg außen entlangführt. Das Ventilorgan weist ein offenes Ende auf, das in den Auslaßkanal hineinragt. Wenn der Druck in der Ventilkammer einen Grenzwert übersteigt, strömt das Fluid an der Außenseite des Ventilorgans entlang, das dadurch von der Wand des Auslaßkanals abhebt. Der Öffnungsdruck des Rückschlagventils kann durch geeignete Wahl des Materials oder der Wandstärke des Ventilorgans eingestellt werden. Es besteht daher die Möglichkeit, mehrere Ventilorgane von unterschiedlicher Steifigkeit bereitzuhalten und davon eines in dem Rückschlagventil zu montieren.

Das erfindungsgemäße Rückschlagventil eignet sich besonders für künstliche Harnblasen. Diese benötigen einlaßseitig ein Rückschlagventil mit geringem Öffnungsdruck und auslaßseitig ein Rückschlagventil mit höherem Öffnungsdruck. Für beide Rückschlagventile kann jeweils das gleiche Gehäuse, jedoch mit unterschiedlichen Ventilorganen, benutzt werden.

Das Rückschlagventil eignet sich generell als Implantat oder für die Verwendung bei Implantaten. Es wird hierzu vorzugsweise aus einem Silikon hergestellt. Da Silikone nicht schweißbar sind, wird das Ventil aus zwei vorgefertigten Formkörpern zusammengesetzt, wobei das Ventilorgan an einem dieser Formkörper durch Schweißen, Vulkanisierung oder Klemmung befestigt wird.

Das Rückschlagventil ist nicht auf Implantate oder auf den medizinischen Bereich beschränkt. Es kann überall dort eingesetzt werden, wo ein definiertes Öffnungsverhalten erforderlich ist, z.B. auch bei Infusionssystemen.

Im folgenden werden unter Bezugnahme auf die Zeichnungen Ausführungsbeispiele der Erfindung näher erläutert.

Es zeigen:
- Fig. 1: die Darstellung einer künstlichen Harnblase im implantierten Zustand,
- Fig. 2: einen Längsschnitt durch zwei ausgangsseitig miteinander vereinigte Rückschlagventile des Blasensystems der Fig. 1,
- Fig. 3: ein weiteres Ausführungsbeispiel des in ein Leitungssystem integrierten implantierbaren Rückschlagventils, und
- Fig. 4: einen Ureter-Bypass unter Verwendung der Rückschlagventile.

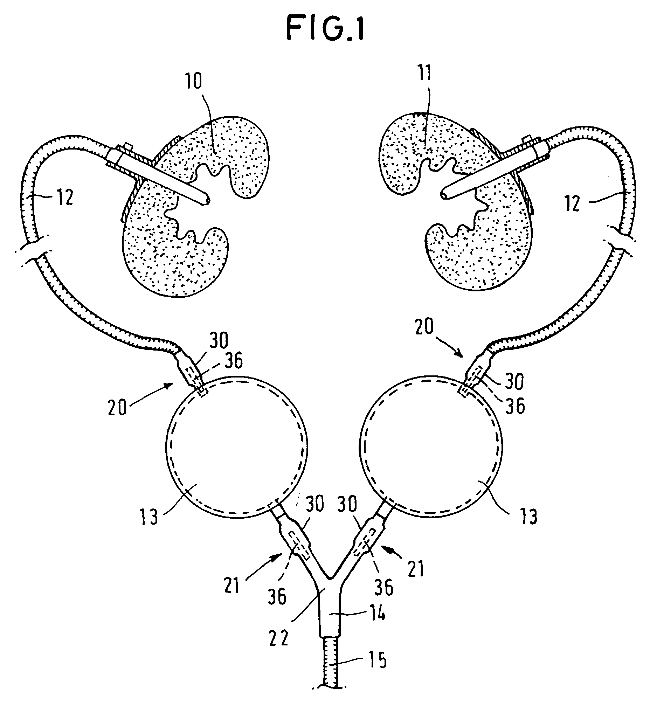

In Fig. 1 ist ein künstliches Blasensystem dargestellt, das in einen Patienten implantiert ist, um Harn aus den Nieren 10,11 abzuleiten. Zu diesem Zweck ist in jede Niere ein Harnleiterkatheter 12 eingeführt, dessen anderes Ende mit einer künstlichen Blase 13 verbunden ist. Bei dem vorliegenden Ausführungsbeispiel sind zwei künstliche Blasen (für jede Niere 10,11 eine) vorgesehen. Jede dieser Blasen besteht aus einem elastischen rückstellfähigen Behälter, der im zusammengedrückten Zustand bestrebt ist, sich aufzuweiten und dadurch über den Katheter 12 Flüssigkeit anzusaugen. Die Blasen 13 werden in der Bauchgegend des Patienten unter der Haut implantiert, wobei der Patient von außen mit der Hand gegen die Blasen drücken kann, um diese zu entleeren. Ausgangsseitig sind die Blasen 13 mit einem Y-Stück 14 verbunden, das in eine künstliche oder natürliche Harnröhre 15 einmündet, die von dem Schließmuskel oder einem externen Sphinkter abgedrückt oder freigegeben wird.

Jede Blase 13 weist an ihrem Einlaßende ein erstes Rückschlagventil 20 und an ihrem Auslaßende ein zweites Rückschlagventil 21 auf. Das erste Rückschlagventil 20 ist in Richtung zur Blase 13 hin offen, während das zweite Rückschlagventil 21 in Richtung aus der Blase heraus offen ist. Beide Rückschlagventile 20,21 sind normalerweise gesperrt und sie öffnen erst bei Erreichen eines bestimmten Öffnungsdrucks. In Gegenrichtung sind sie ständig gesperrt.

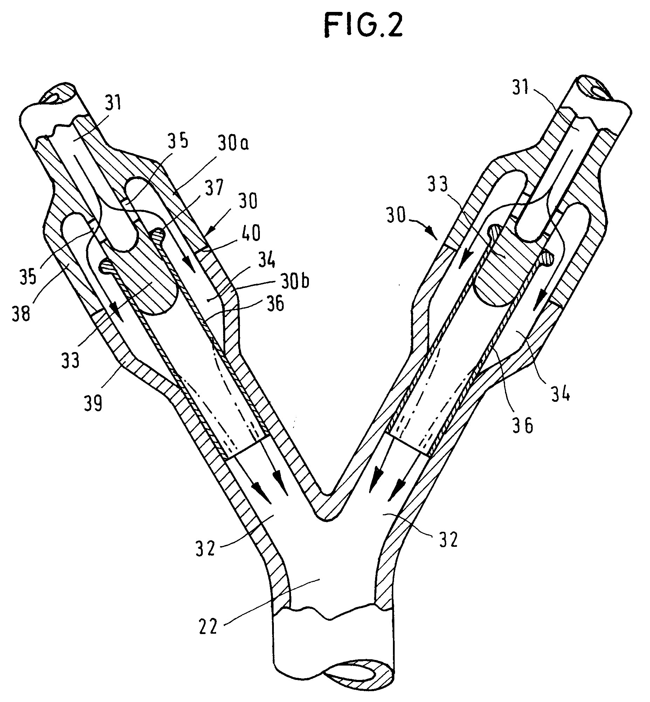

Fig. 2 zeigt die beiden an die Auslässe der Blasen 13 angeschlossenen Rückschlagventile 21, deren Auslaßkanäle in dem Y-Stück 22 vereinigt sind. Die beiden Rückschlagventile 21 sind untereinander gleich ausgebildet. Jedes Rückschlagventil weist ein rohrförmiges Gehäuse 30 auf, das an einem Ende mit einem Einlaßkanal 31 und am anderen Ende mit einem Auslaßkanal 32 einstückig verbunden ist. Der Durchmesser des Gehäuses 30 ist etwas größer als der Außendurchmesser von Einlaßkanal 31 und Auslaßkanal 32. Der Einlaßkanal 31 führt in einen koaxial hierzu verlaufenden Blindstutzen 33 hinein, der sich in eine Ventilkammer 34 im Innern des Gehäuses 30 erstreckt. Der Blindstutzen 33 ist mit seitlichen Öffnungen 35 versehen, die den Einlaßkanal 31 mit der Ventilkammer 34 verbinden. Der Einlaßkanal 31 endet im Innern des Blindstutzens 33, welcher an seinem Ende verschlossen ist.

Auf den Blindstutzen 33 ist ein schlauchförmiges Ventilorgan 36 aufgeschoben, das aus einem elastischen formstabilen Schlauch aus Kunststoffolie besteht. Dieser Schlauch sitzt mit Spannung auf dem Blindstutzen 33. Er weist an seinem Ende einen Ringwulst 37 auf, der das Festhalten auf dem Blindstutzen begünstigt. Alternativ oder zusätzlich kann der Schlauch an dem Blindstutzen verklebt sein.

Das schlauchförmige Ventilorgan 36 ist (mit Ausnahme des Blindstutzens 33) hohl und leer und es ragt über die Ventilkammer 34 hinaus ein Stück weit in den Auslaßkanal 32 hinein. Der Außendurchmesser des zylindrischen Ventilorgans 36 ist so bemessen, daß das Ventilorgan mit leichter Spannung gegen die Wand des Auslaßkanals 32 drückt und somit den Fluidfluß aus der Ventilkammer 34 heraus abdichtet. Das Ventilorgan 36 ragt lose in den Auslaßkanal 32 hinein und ist mit diesem nicht fest verbunden.

Das Gehäuse 30 setzt sich aus zwei Gehäuseteilen 30a,30b zusammen. Jedes dieser Gehäuseteile weist eine Ringwand 38 bzw. 39 auf. Diese Ringwände 38,39 sind an einer Verbindungsstelle 40 miteinander verbunden und sie umschließen die Ventilkammer 34. Die Ringwand 38 ist einstückig mit der Wand des Einlaßkanals 31 und dem Blindstutzen 33 geformt. Die Ringwand 39 ist einstückig mit der Wand des Auslaßkanals 32 geformt.

Das Gehäuse 30 und das Ventilorgan 36 bestehen vorzugsweise aus Silikonmaterial, das von hoher Körperverträglichkeit ist. Das Gehäuse 30 ist im wesentlichen starr, während das Ventilorgan 36 ein elastischer formstabiler Schlauch ist. Die Wandstärke dieses Schlauchs wird entsprechend dem gewünschten Öffnungsdruck gewählt. So haben beispielsweise die Rückschlagventile 20 an der Einlaßseite der Blase 13 einen Öffnungsdruck von 0,5 cm Wassersäule, während die Rückschlagventile 21 auf der Auslaßseite einen Öffnungsdruck von 25 cm Wassersäule haben. Obwohl die Ventile 20 und 21 generell den gleichen Aufbau haben, unterscheiden sie sich durch das jeweils benutzte Ventilorgan 36.

Wenn der Öffnungsdruck in der Ventilkammer 34 den jeweiligen Grenzwert übersteigt, hebt die Wand des Ventilorgans 36 von derjenigen des Auslaßkanals 32 ab und das Ventil wird durchlässig. In Gegenrichtung ist das Ventil stets undurchlässig, da ein Druck am Auslaßkanal 32 das Ventilorgan 36 aufweitet und noch fester gegen die Wand des Auslaßkanals drückt.

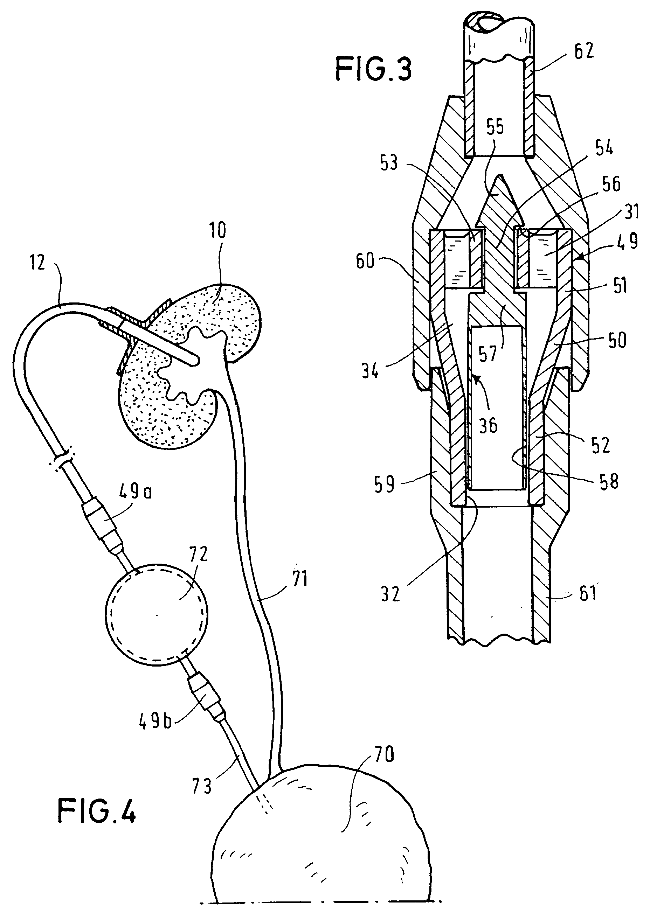

Das Rückschlagventil 49 von Fig. 3 weist ein starres Ventilgehäuse 50 auf, das im wesentlichen rohrförmig ist und einen Bereich größeren Durchmessers 51 und einen Bereich kleineren Durchmessers 52, welcher den Auslaßkanal 32 bildet, aufweist. Im Bereich größeren Durchmessers 51 befindet sich ein Ring 53, der von einem Armstern gehalten wird. Der Ring 53 dient als Halter für das Ventilorgan 36. Das Ventilorgan 36 besteht aus einem einstückigen Körper aus weichelastischem Kunststoff, der jedoch unterschiedliche Wandstärken hat. Es weist einen relativ dicken Stiftteil 54 auf, der in einer Spitze 55 mit Hinterschneidung 56 endet, und einen Halteteil 57, dessen Durchmesser größer ist als der Innendurchmesser des Ringes 53. Ebenso wie der Stiftteil 54 ist auch die Spitze 55 und der Halteteil 57 massiv ausgebildet. Von dem Halteteil 57 steht eine zylindrische dünnwandige Schürze 58 axial ab. Diese Schürze 58 bildet das eigentliche Ventilelement, das sich passend an die Wand des Auslaßkanals 32 anlegt. Es bildet ein weiches ganz leicht verformbares Häutchen.

Das Ventilgehäuse 50 wird auslaßseitig von einer Muffe 59 und einlaßseitig von einer Muffe 60 der abgehenden Schlauchleitung 61 bzw. der ankommenden Schlauchleitung 62 teilweise übergriffen. Die Muffe 60 überlappt die Muffe 59 und beide Muffen 59,60 umschließen das Ventilgehäuse 50 passend und eng. Im Überlappungsbereich sind die Muffen 59,60 miteinander verklebt oder verschweißt. Die Schlauchleitungen bestehen aus körperverträglichem Kunststoff, vorzugsweise aus Silikonmaterial.

In Figur 4 ist ein Ausführungsbeispiel dargestellt, bei dem das Rückschlagventil im Rahmen eines Ureter-Bypasses benutzt wird. Der Ureter ist der Harnleiter 71, der von der Niere 10 zu der natürlichen Blase 70 führt. Dieser Harnleiter ist peristaltisch, d.h. er pumpt durch Muskelbewegungen den Urin von der Niere zur Blase. Wenn der Harnleiter durch einen Tumor komprimiert wird oder aus anderen Gründen seine Funktion nicht wahrnehmen kann, wird ein Harnleiterkatheter 12 in die Niere eingeführt, welcher subkutan mit einer implantierten Kunstblase 72 verbunden ist. Vom Auslaß der Kunstblase 72 führt ein Schlauch 73 in die natürliche Blase 70. Am Einlaß der Kunstblase 72 befindet sich ein Rückschlagventil 49a und am Auslaß befindet sich ein Rückschlagventil 49b. Die Kunstblase 72 ist eine selbstrückstellende ansaugende Blase, die Urin aus der Niere 10 ansaugt. Durch manuellen Druck auf die Kunstblase 72 kann diese durch das Rückschlagventil 49 in die Blase 70 hinein entleert werden. Die manuell ausgeübte Pumpwirkung an der Kunstblase 72 ersetzt somit die peristaltische Pumpbewegung des Harnleiters 71.

Bei dem Ureter-Bypassystem von Figur 4 ist der Eingriff in den Patientenkörper nicht so groß, wie beim Enfernen der natürlichen Blase.

## Patentansprüche

1. Rückschlagventil, insbesondere für eine implantierbare künstliche Harnblase, mit einem Einlaßkanal (31), einem Auslaßkanal (32) und einer dazwischen angeordneten Ventilkammer (34), die ein schlauchförmiges Ventilorgan (36) mit verformbarem Querschnitt enthält,
**dadurch gekennzeichnet**,
daß das Ventilorgan (36) ein geschlossenes Ende und ein offenes Ende aufweist, daß das geschlossene Ende des Ventilorgans (36) in der mit dem Einlaßkanal (31) in offener Verbindung stehenden Ventilkammer (34) angeordnet ist, und daß das offene Ende des Ventilorgans (36) mit seiner Außenseite an der Wand des Auslaßkanals (32) anliegt, wobei im Falle des Überschreitens des Öffnungsdrucks das Ventilorgan (36) von der Wand des Auslaßkanals (32) abhebt.

2. Rückschlagventil nach Anspruch 1, dadurch gekennzeichnet, daß von der Wand des Einlaßkanals (31) ein hohler Blindstutzen (33) axial absteht, der mindestens eine in die Ventilkammer (34) führende Öffnung (35) aufweist und das eine Ende des schlauchförmigen Ventilorgans (36) verschließt.

3. Rückschlagventil nach Anspruch 1 oder 2, dadurch gekennzeichnet, daß ein Gehäuse (30) mit zwei Gehäuseteilen (30a,30b) vorgesehen ist, von denen das erste Gehäuseteil (30a) den Einlaßkanal (31), einen den Einlaßkanal verlängernden hohlen Blindstutzen (33) mit mindestens einer seitlichen Öffnung (35) und eine den Blindstutzen umgebende erste Ringwand (38) aufweist, und von denen das zweite Gehäuseteil (30b) den Auslaßkanal (32) und eine daran anschließende erweiterte zweite Ringwand (39) aufweist, die mit der ersten Ringwand (38) passend verbunden ist.

4. Rückschlagventil nach Anspruch 2 oder 3, dadurch gekennzeichnet, daß das schlauchförmige Ventilorgan (36) auf den Blindstutzen (33) aufgeschoben und an diesem fixiert ist.

5. Rückschlagventil nach Anspruch 1, dadurch gekennzeichnet, daß in einem im wesentlichen rohrförmigen Ventilgehäuse (50) der Einlaßbereich einen koaxialen Ring (53) enthält, der ein stiftförmiges Halteteil (54) des Ventilorgans 36 umgibt.

6. Rückschlagventil nach einem der Ansprüche 1-5, dadurch gekennzeichnet, daß zwei Ventile zu einer Y-Struktur zusammengefaßt sind, wobei die Auslaßkanäle (32) der Ventile verbunden sind.
